# EUROPEAN PATENT APPLICATION

(11) **EP 2 368 456 A2**
(43) Date of publication of application: **28.09.2011**
(21) Application number: 11382070.8
(22) Date of filing: 15.03.2011
(51) Int. Cl.: A45D 34/02, A45D 34/04

(54) **Device for testing scents**

(30) Priority: 24.03.2010 ES 201030435
(71) Applicant: Planet Gratovil, Quirze, 08391 Tiana Barcelona (ES)
(72) Inventor: Planet Gratovil, Quirze, 08391 Tiana Barcelona (ES)
(74) Representative: ABG Patentes, S.L.

(57) **Abstract**

The present invention relates to a device which allows testing scents such that it gives off an amount of scent when the user wishes to test it.

Another object of the invention is the possibility of readily changing the unit of the device for testing scents either when the source of the scent has run out or when the scent is to be changed.

The configuration of the device which allows testing scents according to the invention is intended, among other objectives, to be long-lasting as a result of its structure.

## Description

### Object of the Invention

The present invention relates to a device which allows testing scents such that it gives off an amount of scent when the user wishes to test it.

Another object of the invention is the possibility of readily changing the unit of the device for testing scents either when the source of the scent has run out or when the scent is to be changed.

The configuration of the device which allows testing scents according to the invention is intended, among other objectives, to be long-lasting as a result of its structure.

### Background of the Invention

Testing scents coming from a source of a scent requires using means which keep the scent stored away and dispense it only when there is a desire to smell it, i.e., when it is tested.

The simplest case is the use of perfume or cologne sprayers in which the source of the scent is a liquid with fragrance. The liquid is stored in a deposit and is dispensed by spraying when the scent is tested.

Dispensers of this type spray liquid, generating drops having a characteristic diameter, endeavoring that they are as small as possible to favor evaporation because scents are perceived when said evaporation has taken place.

The evaporation process brings about changes in perceived scents because the evaporation of different volatile substances does not always occur at the same time, and as a result they are not perceived at the same concentrations either.

An alternative to apparatuses of this type are devices for testing scents having a container or cavity where the source of the scent is located, and where there is also air to favor the mixture before being dispensed to test the scent.

European patent with publication number EP1259136, which discloses a scent dispenser based on using a closed bellows is known. The source of the scent is placed inside the bellows and the latter is closed. This bellows can be located inside a casing to favor handling and dispensing.

In operation, both the source of the scent and a certain amount of air are located inside the bellows such that the volatile substances responsible for the scent mix with the air. When the scent is to be tested, the user presses the bellows creating an internal pressure that is greater than the external pressure. The bellows has outlets formed by valves such that given the pressure difference the scent comes out through the valves in order to be perceived.

The use of bellows requires using flexible materials given that the operation requires the deformation of the material forming the bellows. Particularly, permanent local deformations and stresses that are greater than those in the rest of the structure are generated in the folds which give way to the bellows-shaped structure, generating damage which worsens with use until finally breaking. When any break occurs, when the user exerts force, increasing the internal pressure, the air comes out through said break instead of through the valve because the resistance to air going out is less in the break than in the valve. Given that a bellows has a very considerable length of folds as a result of multiplying the perimeter of the fold by the number of folds, the probability of breaking and being rendered useless is also very high.

The present invention according to claim 1 provides a device structure for testing scents which avoids the use of bellows and has a greater resistance to use and durability. Other additional advantages are obtained by means of the features described in the remaining claims.

### Description of the Invention

The present invention consists of a device for testing scents comprising a set of components operating with one another to robustly allow dispensing a mixture of air with the volatile substances coming from the source of the scent. This set of components is formed at least by:
- *a container for storing the source of the scent,*
   The source of the scent can be an absorbent material or liquid that has been provided with an amount of perfume, both considered only as examples. An enclosure in the lower part of the container serving as a storage site of said source will be shown in the example described below as an embodiment of the invention.
- *a cap which is coupled* to *the container such that both bodies, the container and the cap, create an internal space for retaining the scent coming from the source of the scent,*
   This internal space is where the volatile substances coming from the source of the scent mix with the enclosed air. The cap closes the enclosure and, together with the container, give rise to what is referred to hereinafter as a unit for dispensing the scent. In the example described below, both the cap and the container comprise more than one piece, allowing in this particular case the manufacture by means of injection molds without these molds requiring complicated moving parts which make manufacture more expensive.
- *guiding means between the cap and the container such that the cap presents at least axial displacement with respect* to *the container,* where *this axial displacement is limited in a determined range,*
   The cap is moveable with respect to the container. Since it presents at least axial movement, the inner volume enclosed by the cap together with the container varies with this axial movement. When both pieces are brought closer to one another, the volume is reduced and forces the scent to go out; and when they are separated from one another, the entrance of air is favored, so that such air mixes with the volatile substances that continue to be given off by the source of the scent for later testing.
   The guiding means between the cap and the container form a range of displacement, a first limit of this range corresponds to the limit in the displacement due to the force exerted on the cap to force the scent to go out and, a second limit, which corresponds to the maximum separation between the cap and the container after recovering the original position when the user releases the cap after having exerted force to test the scent.
- *a spring arranged in the internal space such that one end of the spring is supported on the cap and the other end of the spring is supported on the container,* where *said spring maintains a bias between the cap and the container* to *be spaced from one another,*
   This spring is responsible for the positional recovery of the cap after being pressed by the user. The user presses and forces the scent to go out. When the user releases the cap, the spring forces the recovery displacement to the limit established by the guiding means.
- *wherein the cap has one* or *more outlets communicating the internal space with the outside of the device such that, in operation, when the cap is pressed against the container, overcoming the resistance of the spring, the internal space is reduced, forcing the scent* to *go out through the outlet* or *outlets,*
   Due to the reduction of the volume defined between the two main components of the unit because of the force exerted on the cap against the resistance of the spring, the outlets allow the scent to go out. These outlets are small in the preferred example such that they retain the scent therein until the device is used without the need for valves.

Dependent claims 2 to 11 relate to embodiments of the invention according to different constructive solutions in which such embodiments are considered to be incorporated herein by reference.

These embodiments include the constructive details which connect the unit with an additional piece referred to as the base and allow fixing and replacing the unit either because the scent is to be changed or because the source of the scent has run out.

Claims 12 to 14 comprise embodiments corresponding to devices provided with this technical solution of a connection with the base to allow the replacement, concerning simply scent dispensing units which also require being pressed to release the scent. Such embodiments are also incorporated herein by reference.

### Description of the Drawings

These and other features and advantages of the invention will become clearer from the following detailed description of a preferred embodiment given only by way of illustrative and nonlimiting example with reference to the attached figures.
Figures 1a and 1b show the device according to an embodiment of the invention in an elevational cross-section view and in an extended position; i.e., in Figure 1a the moveable pieces (container and cap) are separated as much as possible, and in Figure 1b they are in a close position as obtained by means of a force which brings them closer together to release the scent.
Figures 2a and 2b show the same embodiment as in the previous figures with a perpendicular section of the elevational view with respect to the section of Figures 1a and 1b to allow distinguishing in another angular position the local existence of the attachment means between the unit and the base.
Figures 3a, 3b and 3c show three different positions of the same embodiment in a perspective view, one top perspective view and two bottom perspective views. In the bottom view 3c the base has been eliminated in order to see flanges for fixing with the base.
Figures 4a and 4b show an exploded perspective view of the same embodiment, views being both from a lower position and from an upper position.

### Detailed Description of the Invention

The present invention consists of a device for testing scents in which when the user wants to perceive the scent, exerts force and forces the release of the scent. The scent stored in the device and released with this pressure is already mixed with air; and when the user stops exerting force, the positional recovery of the device and the entrance of fresh air for mixing with more volatile substances being released in the source of the scent arranged inside the device are favored.

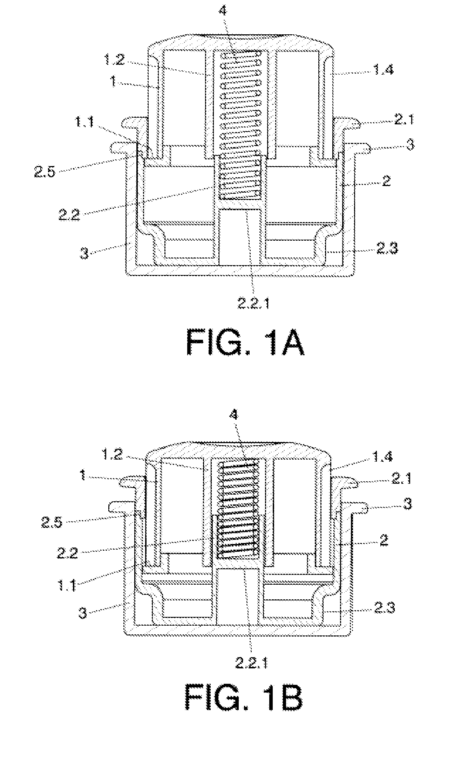

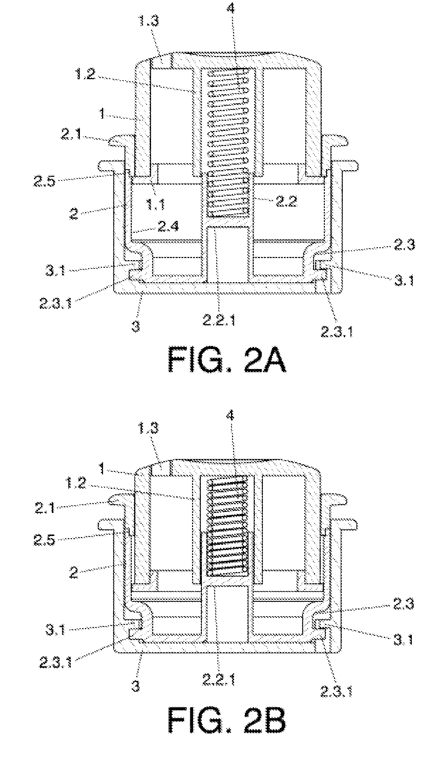

Figures 1 to 4, with all their variants, show one and the same embodiment that will be used to describe different constructive solutions, views 1 and 2 being sectional views in which the internal structure is more clearly seen.

The device according to the embodiment consists of two sets of pieces associated with a container (2) and with a cap (1). The embodiment, being a particular manner of carrying out the invention, makes use of a cylindrical configuration in which the container (2) adopts the shape of a cup which allows housing the source of the scent (not graphically depicted for the sake of clarity) in its lower part. This source of the scent can be, for example, a solid absorbent material embedded in a perfume.

The container (2) is laterally and internally provided with a cylindrical guide wall (2.4) which allows guiding the axial displacement of the cap (1).

In this case, the configuration of a cylindrical wall relates to a cylindrical surface having a circular section according to the embodiment. Nevertheless, the term cylindrical must be interpreted in the broadest sense of the word, where a cylindrical surface can be generated by a polygon generator which is displaced along a directrix line. The resulting cylindrical surface would thus have a section transverse to its directrix axis according to a polygon.

The cap (1) in turn has a closure ring (1.1) formed in this embodiment by a second piece that can be fitted on the main body of the cap (1). The closure ring (1.1) has a shape that fits the guide wall (2.4) of the container (2), establishing a closure to prevent the scent from coming out through this fitting, while at the same time allowing the axial displacement and guiding between both pieces: the cap (1) and the container (2).

Given that this embodiment has been carried out in plastic and by means of an injection method, the embodiment of the cap (1) in one piece and of the closure ring (1.1) in another different piece allows a simple manufacture which facilitates the release of the pieces from the mold without the closure ring (1.1) preventing demolding.

The cap (1) thus moves axially and it does this between two limits: a lower limit (according to the position shown in the drawings) established by a notched lower area (2.3) of the container (2); and an upper limit due to the presence of a step generated by a smaller diameter also in the upper part of the container (2).

When the two main pieces, the cap (1) and the container (2), are brought closer to one another, the internal space is reduced, forcing the air therein mixed with the scent to tend to come out. In this embodiment the air and scent come out by means of outlets (1.3) located in the upper surface of the cap (1).

This smaller diameter in the upper part of the container (2) also hinders manufacture by means of injection molding such that it has been obtained in this embodiment by means of using two pieces: the main body of the container (2) and a retaining ring (2.1) which fits in a seating (2.5) of the main body of the container (2) located on its upper edge.

The cap (1) and the container (2) provide an internal space which is what allows the mixture of the scent and the air. An internal spring (4) maintains the bias of both components to be separated from one another.

Both the container (2) and the cap (1) have guides (1.2, 2.2) for the spring (4): an upper spring guide (1.2) and lower spring guide (2.2). In this embodiment such guides (1.2, 2.2) are formed by cylindrical sectors housing the ends of the spring (4). It can be seen in the figures that contain a section, that one cylindrical sector has a smaller diameter than the other so that when they are brought closer to one another due to the effect of the pressure of the user when forcing the scent to come out, one sector is housed inside the other and the distance whereby they can be brought closer to one another increases with the compression of the spring (4).

Additionally, a raised support (2.2.1) reducing the length of the spring (4) has been incorporated in the sector of the lower guide (2.2) for the spring (4) so that the latter does not have the complete height of the unit.

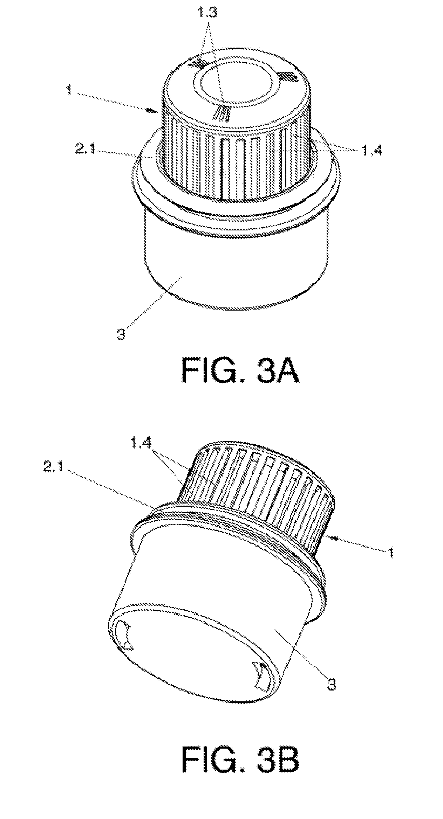

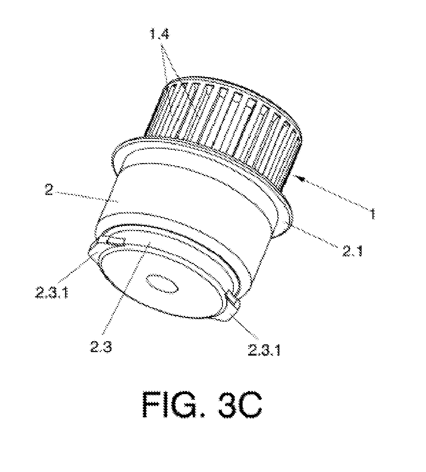

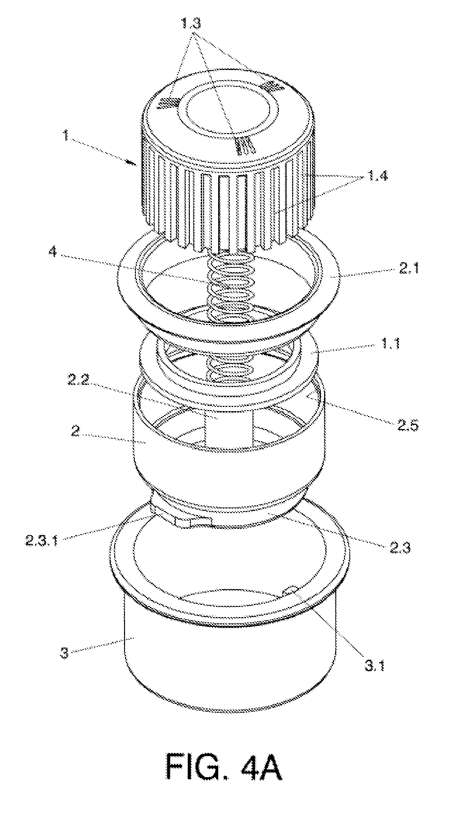

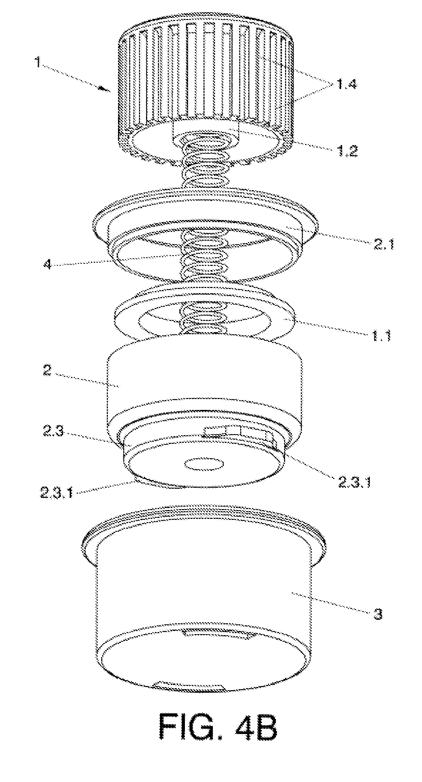

In relation to Figures 1a, 1b, 3c and the exploded perspective views of Figures 4a and 4b, it can be observed that the unit formed by the container (2) and the cap (1) has coupling means for being able to fix said unit to a base (3).

In this embodiment, the coupling means comprise flanges (2.3.1) arranged on the outer surface of the container (2) intended to enter a retaining housing (3.1) arranged in the base (3) .

The flanges (2.3.1) are located in a notched lower area (2.3) of the container (2) providing a smaller diameter in the lower part. This notched lower area (2.3) allows protecting the set formed by the flange (2.3.1) and the retaining housing (3.1) arranged in the base (3).

This retaining housing (3.1) allows the entrance of the flange (2.3.1) by means of a rotating movement of the unit with respect to the base (3), and it has an end stop for said rotation. The connection of the unit and the base (3) is thus carried out by axially introducing the unit without the flange (2.3.1) coinciding with the housing (3.1) to subsequently achieve the final fixing by means of rotating until locating the stop.

The rotation of the unit is facilitated in this embodiment by means of the presence of external vertical grooves (1.4) present on the cap (1).

## Claims

1. A device for testing scents comprising:
• a container (2) for storing the source of the scent,
• a cap (1) which is coupled to the container (2) such that both bodies, the container (2) and the cap (1), create an internal space for retaining the scent coming from the source of the scent,
• guiding means (2.4, 1.1) between the cap (1) and the container (2) such that the cap (1) presents at least axial displacement with respect to the container (2), where this axial displacement is limited in a determined range,
• a spring (4) arranged in the internal space such that one end of the spring (4) is supported on the cap (1) and the other end of the spring (4) is supported on the container (2), where said spring (4) maintains a bias between the cap (1) and the container (2) to be spaced from one another; and, wherein the cap (1) has one or more outlets (1.3) communicating the internal space with the outside of the device such that, in operation, when the cap (1) is pressed against the container (2), overcoming the resistance of the spring (4), the internal space is reduced, forcing the scent to go out through the outlet or outlets (1.3).

2. The device according to claim 1, **characterized in that** the guiding means (2.4, 1.1) between the cap (1) and the container (2) comprise:
• a closure ring (1.1) arranged in the cap (1),
• an internal cylindrical wall (2.4) of the container (2), wherein the closure ring (1.1) is supported on the internal wall (2.4) of the container (2), allowing the axial displacement of one with respect to the other.

3. The device according to claim 2, **characterized in that** the internal wall (2.4) is limited by a step generated by one of the axial displacement limits.

4. The device according to claims 1 and 2, **characterized in that** the cap (1) and the closure ring (1.1) present in the cap (1) are separate pieces that can be coupled to one another.

5. The device according to claims 1 to 3, **characterized in that** the step generated by the axial displacement limit is formed by means of a retaining ring (2.1) that can be coupled to the container (2) such that the internal diameter of the retaining ring (2.1) is smaller than the internal diameter of the cylindrical internal wall (2.4) of the container.

6. The device according to claim 1, **characterized in that** the spring (4) is supported inside two essentially cylindrical sectors, a first sector integral with the cap (1) creating an upper guide (1.2) for the spring (4) and a second sector integral with the container (2) creating a lower guide (2.2) for the spring (4).

7. The device according to claim 6, **characterized in that** one of the upper or lower guides (1.2, 2.2) for the spring (4) has a support (2.2.1) displaced with respect to the base where the guide (1.2, 2.2) is located.

8. The device according to claim 1, **characterized in that** it comprises a base (3) and coupling means for coupling with said base (3) to allow replacement.

9. The device according to claim 8, **characterized in that** the coupling means between the device and the base (3) comprise at least:
• a flange (2.3.1) located in the outer surface of the container (2),
• a retaining housing (3.1) for the flange (2.3.1) arranged in the base (3).

10. The device according to claim 9, **characterized in that** the retaining housing (3.1) for the flange (2.3.1) allows the entrance of the flange (2.3.1) by means of a rotating movement of the container (2) with respect to the base (3), and it has an end stop for said rotation.

11. The device according to any of claims 8 to 10, **characterized in that** the container (2) has a setback region to make room for the coupling means for coupling with the base (3).

12. The device for testing scents comprising:
• a unit for storing the source of the scent in an internal space, with one or more outlets (1.3) communicating the internal space with the outside of the unit such that, in operation, when said unit is pressed, the internal space is reduced, forcing the scent to go out through the outlet or outlets (1.3),
• a base (3),
• coupling means between the base (3) and the unit for storing the source of the scent allowing the replacement of the unit.

13. The device according to claim 12, **characterized in that** the coupling means between the device and the base (3) comprise at least:
• one flange (2.3.1) located on the outer surface of the unit,
• one retaining housing (3.1) for the flange (2.3.1) arranged in the base (3).

14. The device according to claim 13, **characterized in that** the retaining housing (3.1) for the flange (2.3.1) allows the entrance of the flange (2.3.1) by means of a rotating movement of the unit with respect to the base (3), and it has an end stop for said rotation.
